# EUROPÄISCHE PATENTSCHRIFT

(11) **EP 0 884 034 B1**
(45) Veröffentlichungstag und Bekanntmachung des Hinweises auf die Patenterteilung: **02.04.2003**
(21) Anmeldenummer: 98107069.1
(22) Anmeldetag: 17.04.1998
(51) Int. Cl.: A61F 2/66

(54) **Federelastischer Fusseinsatz**
Elastic spring insert for foot
Insert de ressort élastique pour pied

(30) Priorität: 24.04.1997 DE 29707416 U
(43) Veröffentlichungstag der Anmeldung: 16.12.1998
(73) Patentinhaber: Otto Bock HealthCare GmbH, 37115 Duderstadt (DE)
(72) Erfinder: Mosler, Lüder, Dr. Ing., 37115 Duderstadt (DE); Pusch, Martin, Dipl.-Ing., 37115 Duderstadt (DE)
(74) Vertreter: Gramm, Werner, Prof. Dipl.-Ing.

(56) Entgegenhaltungen:
- WO-A-93/04645
- DE-A- 4 205 900
- US-A- 3 833 941
- US-A- 5 139 525
- US-A- 5 549 711

## Beschreibung

Die Erfindung betrifft einen federelastischen Fußeinsatz für einen Kunstfuß, mit zumindest einer Blattfeder.

Federelastische Fußeinsätze sind offenbart z. B. in der US-A-5, 549 711 US-A-4,959,073, in der DE 40 38 063 C2 und DE 42 05 900 A1, in der FR-A1-26 40 499 sowie in dem deutschen Gebrauchsmuster G 93 15 665.0. Die hier eingesetzten Blattfedern sind einer außerordentlich hohen Belastung ausgesetzt. Dabei können die verwendeten Federn aus Carboncomposit, aus Titan oder auch anderen geeigneten Materialien gefertigt sein. Die funktionell erforderliche Verformung führt zu hohen Spannungen, zu deren Aufnahme die Dauerfestigkeit der verwendeten Blattfedern häufig nicht ausreicht.

Der Erfindung liegt die Aufgabe zugrunde, die Strukturfestigkeit der in federelastischen Fußeinsätzen verwendeten Blattfedern zu erhöhen.

Diese Aufgabe wird erfindungsgemäß dadurch gelöst, daß die zumindest eine Blattfeder aus zumindest zwei parallel geschalteten Blattfederelementen besteht, die nebeneinander angeordnet und in ihren beiden Endbereichen miteinander verbunden sind und zwischen diesen beiden Endbereichen einen lichten Abstand voneinander aufweisen, wobei die Verbindung in zumindest einem der beiden Endbereiche momentenstarr ausgebildet ist.

Während eine bloße Materialverstärkung das Problem in der Regel nicht zu lösen vermag, läßt sich die Strukturfestigkeit durch Parallelschaltung von zwei weicheren Federn überraschend wirksam verbessern, ohne hierdurch die erforderliche Federcharakteristik in unerwünschter Weise zu verändern.

Je nach Formgebung und Funktion der Blattfeder ist auf einen ausreichenden lichten Abstand zwischen den beiden Blattfederelementen zu achten, um bei einer Verformung der Blattfeder ein frühzeitiges Anlegen der Blattfederelemente aneinander und damit eine sprunghafte Veränderung der Federcharakteristik zu vermeiden.

Dabei ist es zur Erreichung der geforderten Dauerfestigkeit zweckmäßig, wenn zumindest in einem der beiden Endbereiche zwischen den beiden Blattfederelementen ein Abstandshalter vorgesehen ist.

Zur Beeinflussung der Gesamtsteifigkeit des Fußeinsatzes ist es ferner zweckmäßig, wenn sich die beiden eine Vorfußfeder bildenden Blattfederelemente in dem zwischen ihnen gebildeten lichten Abstandsbereich auf einem Druckpuffer abstützen, der federelastisch oder aber als starrer Abstandshalter ausgebildet sein kann. Dabei ist es vorteilhaft, wenn sich dieser Druckpuffer gegen einen Druckpuffer unterschiedlicher Steifigkeit bzw. unterschiedlicher Federcharakteristik austauschen läßt. Steifigkeit bzw. Federcharakteristik lassen sich aber auch durch Verschiebung eines mechanischen Abstandshalters in Längsrichtung der Blattfederelemente verändern.

Um auf die Steifigkeit Einfluß nehmen zu können, wird erfindungsgemäß der lichte Abstand zwischen den beiden die Vorfußfeder bildenden Blattfederelementen gesteuert. Dabei wird erfindungsgemäß angestrebt, diese Steuerfunktion so zu nutzen, daß die Steifigkeitseinstellung geregelt wird als Funktion der Gangdynamik des Patienten. Erfindungsgemäß kann für den Druckpuffer ein luftbefülltes Druckkissen verwendet werden, das in vollständig befülltem Zustand an den beiden die Vorfußfeder bildenden Blattfederelementen spannungsfrei anliegt also die Blattfedern nicht vorspannt. Um ein derartiges luftbefülltes Druckkissen als steuerbaren Abstandshalter verwenden zu können, ist es zweckmäßig, wenn dieses Druckkissen an den Druckstutzen eines Luftpumpenelementes angeschlossen ist, das zusammen mit dem Fußeinsatz in den Kunstfuß integriert ist und in Abhängigkeit vom Patientengewicht und/oder durch die Patientenaktivität betätigbar ist. Dabei ist es ferner vorteilhaft, wenn das Luftpumpenelement zwischen die Schenkel einer C-förmig ausgebildeten Fersenfeder so eingesetzt ist, daß sein Arbeitshub eine Funktion der elastischen Verformung der Fersenfeder ist. Da die Federwege der Ferse in der Regel deutlich kleiner eingestellt sind als die des Vorfußes, ist der Einfluß des Fersenweges auf das Gangbild geringer. Erfindungsgemäß wird daher die Verformung der Ferse zur Steuerung der Vorfußsteifigkeit verwendet. Dabei kann in der konstruktiven Ausführung das vorstehend erwähnte Luftpumpenelement beim Zusammendrücken der Fersenfeder beaufschlagt werden.

Die Gangdynamik des Patienten ist eine Funktion seiner Tagesform aber auch seiner aktuellen Beschäftigung. Während die Funktion einer Anpassung an die Tagesform durchaus durch ein sukzessive anpassendes System (wie vorstehend beschrieben mit Hilfe eines Luftkissens) denkbar ist, muß die Anpassung an die momentane Beschäftigung (etwa am Arbeitsplatz) teilweise sehr schnell, praktisch von einem Schritt zum nächsten erfolgen. Eine derart schnelle Anpassung kann jedoch ein pneumatisches System kaum leisten.

Um eine schnelle, automatische Anpassung des Prothesenfußes an die aktuelle Gangdynamik des Patienten zu verwirklichen, wird erfindungsgemäß vorgeschlagen, die Längsverschiebung des als starren Abstandshalter ausgebildeten Druckpuffers zwischen den die Vorfußfeder bildenden Blattfederelementen mechanisch aus der Stärke des Fersenauftritts abzuleiten. Eine konstruktive Lösung kann darin bestehen, daß eine C-förmig ausgebildete Fersenfeder mit ihrem oberen Schenkel unter dem hinteren Endbereich der Vorfußfeder mit dieser verbunden und mit ihrem unteren freien Schenkelende über eine Schubstange so mit dem Druckpuffer verbunden ist, daß bei einem vom Patienten bewirkten Zusammendrükken der Fersenfeder die Schubstange den Druckpuffer in Richtung auf den vorderen Endbereich der Vorfußfeder verschiebt. Tritt der Patient bei einem zügigen Schritt fest auf die Ferse, schiebt die Schubstange den Abstandshalter weiter nach vorne. Bei der darauffolgenden Belastung des Vorfußes wird die Position des Abstandshalters fixiert. Der Patient kann dann mittels des steiferen Vorfußhebels einen längeren Schritt ausführen als ihm dies mit einem weicheren Vorfußhebel möglich wäre. Nach Entlastung des Vorfußes entfällt die Fixierung des Abstandshalters und dieser kann in seine Ausgangsposition zurückkehren. Eine derartige Anordnung erlaubt die Adaption der Vorfußfederhärte für jeden einzelnen Schritt, was bei stark variierenden Tätigkeiten von Vorteil ist.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung schematisch dargestellt. Es zeigen:
- **Figur 1**: In Seitenansicht einen Kunstfuß mit einem federelastischen Fußeinsatz bestehend aus einer C-Feder und einer Basis- oder Vorfußfeder;
- **Figur 2**: die C-Feder gemäß Figur 1 in einer Ausführungsform gemäß der Erfindung;
- **Figur 3**: in einer Darstellung gemäß Figur 1 eine abgewandelte Ausführungsform eines sich pneumatisch selbst adaptierenden Kunstfußes;
- **Figur 4**: in einer Darstellung gemäß Figur 3 eine abgewandelte Ausführungsform eines sich mechanisch selbst adaptierenden Kunstfußes;
- **Figur 5**: im Längsschnitt in der Sagittalebene eine Vorfußfeder mit federelastischen Druckpuffern und
- **Figur 6**: in einer Darstellung gemäß Figur 5 eine Vorfußfeder mit manuell längsverschieblich angeordnetem Abstandshalter.

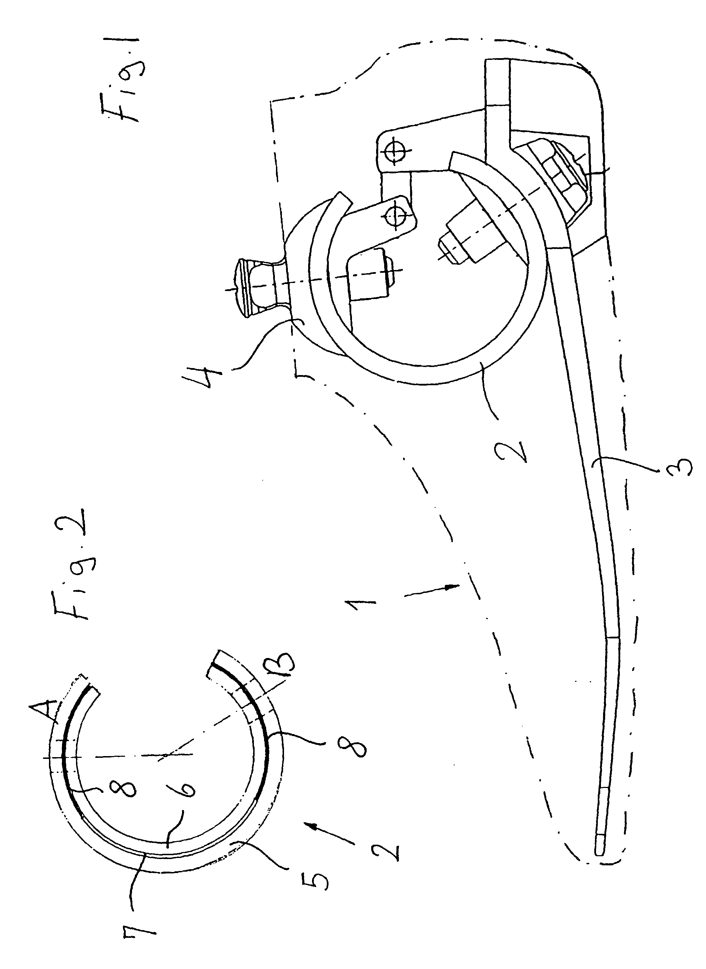

Der in Figur 1 dargestellte gelenklose Kunstfuß weist eine strichpunktiert angedeutete kosmetische Hülle 1 auf, die einen federelastischen Fußeinsatz umschließt. Letzterer setzt sich im wesentlichen zusammen aus einer C-Feder 2, deren unterer Schenkel mit dem hinteren Ende einer Basis- oder Vorfußfeder 3 verschraubt ist. Der obere Schenkel der C-Feder 2 ist mit einem Adapter 4 verschraubt, über den der Kunstfuß an eine Beinprothese anschließbar ist.

Die erfindungsgemäße Ausgestaltung der C-Feder 2 läßt Figur 2 erkennen. Demnach besteht die C-Feder 2 aus zwei parallel geschalteten Blattfederelementen 5, 6, die nebeneinander angeordnet sind, angenähert parallel zueinander verlaufen, in ihren beiden Endbereichen A, B miteinander verbunden sind und zwischen diesen beiden Endbereichen einen lichten Abstand 7 voneinander aufweisen. In diesen beiden Endbereichen A, B ist zwischen den beiden Blattfederelementen 5, 6 jeweils ein Abstandshalter 8 vorgesehen.

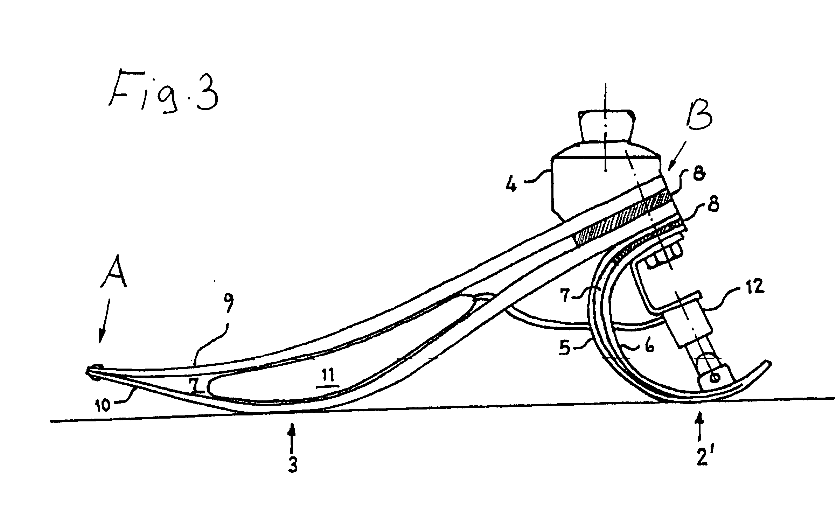

Figur 3 zeigt grob schematisch für den federelastischen Fußeinsatz eine abgewandelte Ausführungsform. Diese umfaßt eine C-förmig ausgebildete Fersenfeder 2', die mit ihrem oberen Schenkel unter dem hinteren Endbereich B einer Vorfußfeder 3 mit dieser verschraubt ist. Die Vorfußfeder 3 setzt sich zusammen aus zwei parallel geschalteten Blattfederelementen 9, 10, die nebeneinander angeordnet und in ihren beiden Endbereichen A, B momentenstarr miteinander verbunden sind. Dabei ist nur im hinteren Endbereich B zwischen den beiden Blattfederelementen 9, 10 ein Abstandshalter 8 vorgesehen. Zwischen den beiden Endbereichen A, B weisen die beiden Blattfederelemente 9, 10 einen lichten Abstand 7 voneinander auf, in den ein federelastischer Druckpuffer 11 eingesetzt ist, der an den beiden Blattfederelementen 9, 10 spannungsfrei anliegt.

Der Druckpuffer 11 ist gemäß dem schematischen Ausführungsbeispiel ein luftbefülltes Druckkissen, das an den Druckstutzen eines Luftpumpenelementes 12 angeschlossen ist, das zusammen mit dem federelastischen Fußeinsatz 2, 3 in den Kunstfuß integriert ist und in Abhängigkeit vom Patientengewicht und/oder durch die Patientenaktivität betätigbar ist. Dabei ist das Luftpumpenelement 12 in diesem Ausführungsbeispiel so zwischen die Schenkel der Fersenfeder 2' eingesetzt, daß bei Fersenbelastung, also beim Zusammendrücken der Fersenfeder 2', das Luftpumpenelement 12 beaufschlagt wird und Luft in den Druckpuffer 11 drückt.

Bei der Ausführungsform gemäß Figur 4 entsprechen die Fersenfeder 2' und die Vorfußfeder 3 im wesentlichen denen der Figur 3. Jedoch ist anstelle einer pneumatischen Adaptierung in Figur 4 eine mechanische Adaptierung dargestellt. Hier ist das untere freie Schenkelende 13 der Fersenfeder 2' über eine Schubstange 14 mit einem einen starren Abstandshalter bildenden Druckpuffer 11' kinematisch verbunden. Das nach vorn weisende Ende der Schubstange 14 ist längsverschieblich in einer seitlich am Druckpuffer 11' sitzenden Kulisse 15 geführt, liegt rückseitig mit einem Mitnehmer 16 an der Kulisse 15 an und stützt sich mit ihrem freien Ende über eine Feder 17 an der Kulisse 15 ab. Tritt der Patient bei einem zügigen Schritt fest auf die Ferse, führt die Verformung der Fersenfeder 2' zu einer Verschiebung der Schubstange 14, die über ihren Mitnehmer 16 den Druckpuffer 11' weiter nach vorne verschiebt, wo er bei der darauffolgenden Belastung des Vorfußes fixiert wird. Die Feder 17 ermöglicht ein Zurückfedern der Fersenfeder 2', ohne hierbei die Position des Druckpuffers 11' zu verändern. Nach Entlastung des Vorfußes entfällt die Fixierung des Abstandshalters, der daraufhin unter Einwirkung der schwach ausgelegten Feder 17 wieder in seine Ausgangsposition zurückkehrt.

Bei diesem Lösungsvorschlag wird somit eine schnelle, automatische Anpassung des Prothesenfußes an die aktuelle Gangdynamik des Patienten verwirklicht, wobei die Steifigkeit der zweilagigen Vorfußfeder mit einem längsverschieblichen, mechanischen Abstandshalter eingestellt wird, dessen Steuerung über die Härte des Fersenauftritts erfolgt.

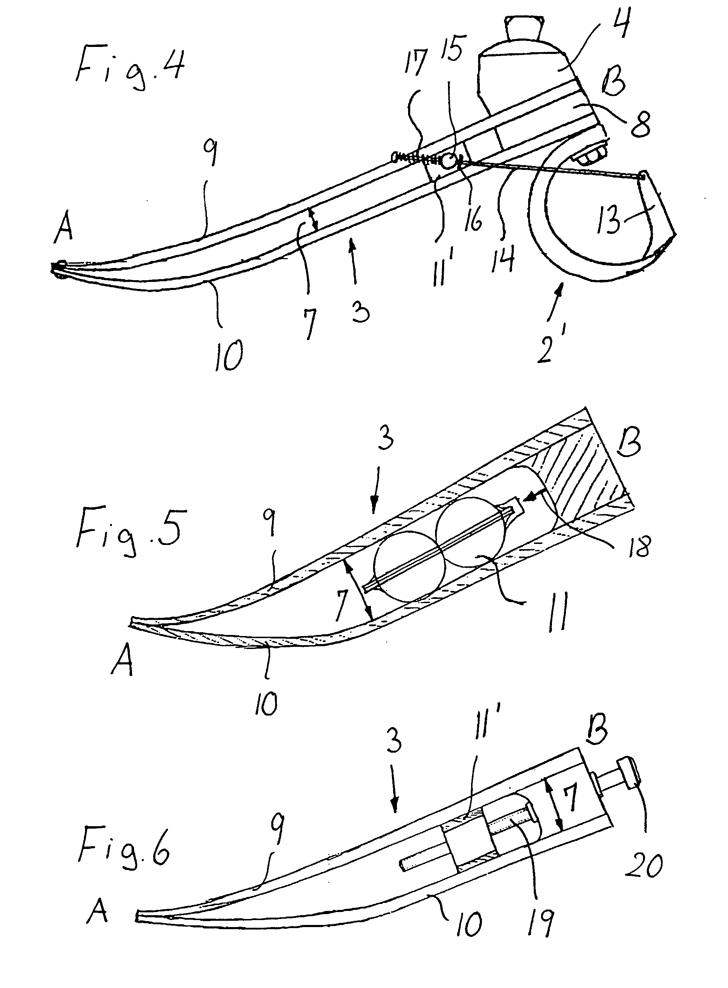

Figur 5 zeigt eine Vorfußfeder 3, die in dem lichten Abstand 7 zwischen ihren beiden Blattfederelementen 9, 10 Druckpuffer 11 aufnimmt, die - wie es der Pfeil 18 andeutet - entweder in Längsrichtung der Vorfußfeder verschiebbar, oder aber in der dargestellten Position komprimierbar sind.

Figur 6 zeigt eine mit Figur 5 vergleichbare Vorfußfeder 3, deren als starrer Abstandshalter ausgebildete Druckpuffer 11' über ein Stellglied 19 längsverschieblich ist. Dieses Stellglied 19 ist als Spindel ausgebildet, auf der der Druckpuffer 11' mit einer nicht näher dargestellten Spindelmutter geführt ist. Die Spindel ragt mit ihrem hinteren Ende aus dem hinteren Endbereich B der Vorfußfeder 3 heraus und kann hier manuell über einen Drehknopf 20 betätigt werden.

## Patentansprüche

1. Federelastischer Fußeinsatz für einen Kunstfuß, mit zumindest einer Blattfeder (2, 3), wobei die zumindest eine Blattfeder (2, 3) aus zumindest zwei parallel geschalteten Blattfederelementen (5, 6; 9, 10) besteht, die nebeneinander angeordnet und in ihren beiden Endbereichen (A, B) miteinander verbunden sind und zwischen diesen beiden Endbereichen einen lichten Abstand (7) voneinander aufweisen, **dadurch gekennzeichnet, daß** die Verbindung in zumindest einem der beiden Endbereiche (A, B) momentenstarr ausgebildet ist.

2. Fußeinsatz nach Anspruch 1, **dadurch gekennzeichnet, daß** zumindest in einem der beiden Endbereiche (A, B) zwischen den beiden Blattfederelementen (5, 6; 9, 10) ein Abstandshalter (8) vorgesehen ist.

3. Fußeinsatz nach Anspruch 1 oder 2, **dadurch gekennzeichnet, daß** sich die beiden eine Vorfußfeder (3) bildenden Blattfederelemente (9, 10) in dem zwischen ihnen gebildeten lichten Abstandsbereich (7) auf einem Druckpuffer (11; 11') abstützen.

4. Fußeinsatz nach Anspruch 3, **dadurch gekennzeichnet, daß** der Druckpuffer (11) federelastisch ausgebildet ist.

5. Fußeinsatz nach Anspruch 3, **dadurch gekennzeichnet, daß** der Druckpuffer (11') als starrer Abstandshalter ausgebildet ist.

6. Fußeinsatz nach Anspruch 3, 4 oder 5, **dadurch gekennzeichnet, daß** der Druckpuffer (11; 11') auswechselbar angeordnet ist.

7. Fußeinsatz nach einem der Ansprüche 3 bis 6, **dadurch gekennzeichnet, daß** der Druckpuffer (11') zwischen den beiden Blattfederelementen (9, 10) in deren Längsrichtung verschiebbar angeordnet ist.

8. Fußeinsatz nach Anspruch 7, **dadurch gekennzeichnet, daß** für die Längsverschiebung des Druckpuffers (11') eine Justiereinrichtung (19, 20) vorgesehen ist, deren manuell zu betätigendes Stellglied (19) aus dem hinteren Endbereich (B) der Vorfußfeder (3) herausragt.

9. Fußeinsatz nach Anspruch 7, **dadurch gekennzeichnet, daß** eine C-förmig ausgebildete Fersenfeder (2') mit ihrem oberen Schenkel unter dem hinteren Endbereich (B) der Vorfußfeder (3) mit dieser verbunden und mit ihrem unteren freien Schenkelende (13) über eine Schubstange (14) so mit dem Druckpuffer (11') verbunden ist, daß bei einem vom Patienten bewirkten Zusammendrücken der Fersenfeder (2') die Schubstange (14) den Druckpuffer (11') in Richtung auf den vorderen Endbereich (A) der Vorfußfeder (3) verschiebt.

10. Fußeinsatz nach Anspruch 4, **dadurch gekennzeichnet, daß** der Druckpuffer (11) ein luftbefülltes Druckkissen ist.

11. Fußeinsatz nach Anspruch 10, **dadurch gekennzeichnet, daß** das luftbefüllte Druckkissen in vollständig befülltem Zustand an den beiden Blattfederelementen (9, 10) spannungsfrei anliegt.

12. Fußeinsatz nach Anspruch 10 oder 11, **dadurch gekennzeichnet, daß** das Druckkissen an den Druckstutzen eines Luftpumpenelementes (12) angeschlossen ist, das zusammen mit dem Fußeinsatz (2', 3) in den Kunstfuß integriert ist und in Abhängigkeit vom Patientengewicht und/oder durch die Patientenaktivität betätigbar ist.

13. Fußeinsatz nach Anspruch 12, **dadurch gekennzeichnet, daß** das Luftpumpenelement (12) zwischen die Schenkel einer C-förmig ausgebildeten Fersenfeder (2') so eingesetzt ist, daß sein Arbeitshub eine Funktion der elastischen Verformung der Fersenfeder (2') ist.

## Claims

1. Resilient foot insert for an artificial foot, having at least one leaf spring (2, 3), the at least one leaf spring (2, 3) consisting of at least two leaf spring elements (5, 6; 9, 10) which are coupled in parallel, are arranged next to one another and are connected to one another at their two end regions (A, B) and, between these two end regions, have a clearance distance (7) from one another, **characterized in that** the connection is designed to be rigid in terms of torque in at least one of the two end regions (A, B).

2. Foot insert according to Claim 1, **characterized in that** a spacer element (8) is provided between the two leaf spring elements (5, 6; 9, 10) at least in one of the two end regions (A, B).

3. Foot insert according to Claim 1 or 2, **characterized in that** the two leaf spring elements (9, 10) forming a forefoot spring (3) are supported on a pressure buffer (11, 11') in the clearance distance (7) formed between them.

4. Foot insert according to Claim 3, **characterized in that** the pressure buffer (11) is designed to be resilient.

5. Foot insert according to Claim 3, **characterized in that** the pressure buffer (11') is designed as a rigid spacer element.

6. Foot insert according to Claim 3, 4 or 5, **characterized in that** the pressure buffer (11; 11') is arranged to be exchangeable.

7. Foot insert according to one of Claims 3 to 6, **characterized in that** the pressure buffer (11') is arranged to be displaceable between the two leaf spring elements (9, 10) in the longitudinal direction thereof.

8. Foot insert according to Claim 7, **characterized in that**, for the longitudinal displacement of the pressure buffer (11'), an adjusting device (19, 20) is provided whose manually operated actuating member (19) projects from the rear end region (B) of the forefoot spring (3).

9. Foot insert according to Claim 7, **characterized in that** a C-shaped heel spring (2') is connected to the forefoot spring (3) via its upper branch under the rear end region (B) of said forefoot spring (3), and, at its lower free branch end (13), is connected to the pressure buffer (11') via a push rod (14) in such a way that when the heel spring (2') is compressed by the patient, the push rod (14) displaces the pressure buffer (11') in the direction towards the front end region (A) of the forefoot spring (3).

10. Foot insert according to Claim 4, **characterized in that** the pressure buffer (11) is an air-filled pressure cushion.

11. Foot insert according to Claim 10, **characterized in that** the air-filled pressure cushion, in the completely filled state, bears without stress on the two leaf spring elements (9, 10).

12. Foot insert according to Claim 10 or 11, **characterized in that** the pressure cushion is connected to the pressure attachments of an air pump element (12) which is incorporated together with the foot insert (2', 3) into the artificial foot and can be actuated as a function of the patient's weight and/or by the patient's activity.

13. Foot insert according to Claim 12, **characterized in that** the air pump element (12) is fitted between the branches of a C-shaped heel spring (2') in such a way that its working stroke is a function of the elastic deformation of the heel spring (2').

## Revendications

1. Insert de pied élastique pour un pied artificiel, comprenant au moins un ressort à lame (2,3), l'au moins un ressort à lame (2,3) se composant d'au moins deux éléments de ressort à lame (5,6; 9,10) montés en parallèle, disposés l'un à coté de l'autre et reliés l'un à autre dans leurs deux régions extrêmes (A, B), et qui sont à une distance libre (7) l'un de l'autre entre ces deux régions extrêmes, **caractérisé en ce qu'**à l'une au moins des deux régions extrêmes (A, B) la liaison est réalisée rigide aux moments.

2. Insert de pied selon la revendication 1, **caractérisé en ce qu'**à au moins une des deux régions extrêmes (A, B) , il est prévu une entretoise (8) entre les deux éléments de ressort à lame (5,6; 9,10).

3. Insert de pied selon la revendication 1 ou 2, **caractérisé en ce que** dans la région de la distance libre (7) constituée entre eux, les deux éléments de ressort à lame (9,10) constituant un ressort d'avant-pied (3) s'appuient sur un tampon presseur (11; 11').

4. Insert de pied selon la revendication 3, **caractérisé en ce que** le tampon presseur (11) est réalisé élastique.

5. Insert de pied selon la revendication 3, **caractérisé en ce que** le tampon presseur (11') est réalisé sous la forme d'une entretoise rigide.

6. Insert de pied selon la revendication 3, 4 ou 5, **caractérisé en ce que** le tampon presseur (11; 11') est monté de façon interchangeable.

7. Insert de pied selon l'une des revendications 3 à 6, **caractérisé en ce que** le tampon presseur (11') est disposé déplaçable entre les deux éléments de ressort à lame (9,10) suivant leur direction longitudinale.

8. Insert de pied selon la revendication 7, **caractérisé en ce que** pour le déplacement longitudinal du tampon presseur (11') il est prévu un dispositif d'ajustement (19,20) dont l'organe de réglage (19) à actionner manuellement sort de la zone extrême arrière (B) du ressort d'avant-pied (3).

9. Insert de pied selon la revendication 7, **caractérisé en ce qu'**un ressort de talon (2') conformé en C, est relié par sa branche supérieure avec le ressort d'avant-pied (3) sous la région extrême arrière (B) de celui-ci, et est relié par son extrémité de branche inférieure libre (13), par l'intermédiaire d'une tige de poussée (14), avec le tampon presseur (11) de telle manière qu'en cas de compression du ressort de talon (2') exercée par un patient, la tige de poussée (14) déplace le tampon presseur (11') en direction de la région extrême avant (A) du ressort d'avant-pied (3).

10. Insert de pied selon la revendication 4, **caractérisé en ce que** le tampon presseur (11) est un coussin de pression rempli d'air.

11. Insert de pied selon la revendication 10, **caractérisé en ce qu'** à l'état complètement rempli le coussin de pression rempli d'air repose sans contrainte contre les deux éléments de ressort à lame (9,10).

12. Insert de pied selon la revendication 10 ou 11, caractérisé en ce le coussin de pression est raccordé au raccord de pression d'un élément de pompage d'air (12) qui est intégré dans le pied artificiel avec l'insert de pied (2',3), et qui peut être actionné en fonction du poids du patient et/ou par l'activité du patient.

13. Insert de pied selon la revendication 12, **caractérisé en ce que** l'élément de pompage d'air (12) est inséré entre les branches d'un ressort de talon (2') conformé en C de telle manière que sa course de travail soit une fonction de la déformation élastique du ressort de talon (2').
